Europäisches Patentamt

European Patent Office

Office européen des brevets

(19)

(11) Publication number: **0 136 524**
**A1**

(12) # EUROPEAN PATENT APPLICATION

(21) Application number: **84110149.6**

(22) Date of filing: **25.08.84**

(51) Int. Cl.⁴: **A 61 F 13/18**

(30) Priority: **30.08.83 JP 160882/83**

(43) Date of publication of application:
**10.04.85 Bulletin 85/15**

(84) Designated Contracting States:
**DE FR GB IT**

(71) Applicant: **ZENMI CO. LTD.**
**210 Torii**
**Kainan Wakayama-Ken(JP)**

(72) Inventor: **Nakano, Tadao**
**No. 207, Torii**
**Kainan Wakayama-ken(JP)**

(74) Representative: **Schüler, Horst, Dr. European Patent**
**Attorney**
**Kaiserstrasse 41**
**D-6000 Frankfurt/Main 1(DE)**

(54) **Sanitary napkin.**

(57) Disclosed is a sanitary napkin including at least an absorbent material for menstrual blood, a blood-impermeable material and a covering material, characterized in that the sanitary napkin is provided with at least one bending groove which, during use, can produce a configuration conforming to the shape of the perineal area of the wearer.

## FIG.I

EP 0 136 524 A1

0136524

## SPECIFICATION

TITLE OF THE INVENTION

Sanitary Napkin

BAKCGROUND OF THE INVENTION

Field of the Invention

This invention relates to a sanitary napkin for use in absorbing and holding blood during menstruation or other uterine bleeding.

Description of the Prior Art

In recent years, sanitary napkins formed by layering an absorbent material, a fluid-impermeable material, a cushionining material and a covering material have been developed (see, for example, U.S. Patent 4,333,464). Such sanitary napkins have found general acceptance because of their excellent ability to absorb menstrual blood and hold the blood once absorbed therein.

However, since most of such sanitary napkins are flat and rectangular in shape, they are hard to place in such a way that, during use, the napkin conforms to the shape of the perineal area (i.e., the anteroposterior curvature of the perineal area and the concave space between the buttocks) of the wearer and comes into close contact therewith. Accordingly, such sanitary napkins often fail to absorb menstrual blood satisfactorily, resulting in the exudation or leakage thereof. In order to solve this problem, it has been proposed to form sanitary napkins into various shapes. However, there is individual variation in the shape of the perineal area of females and, therefore, it has seemed practically impossible from the viewpoint of productivity to manufacture sanitary napkins so as to fit a variety of perineal shapes. In addition, sanitary napkins formed into special shapes are undesirable in that, when used, they may cause reluctance on the part of the user.

SUMMARY OF THE INVENTION

The present invention has succeeded in solving the above-described problems encountered in conventional sanitary napkins by providing such sanitary napkins with at least one bending groove which, during use, can produce a configuration conforming to the shape of the perineal area of the wearer, the bending groove being provided on one or both sides of the layer of the absorbent material for menstrual blood, the integrated layer of the absorbent material and the blood-impermeable material, or the whole structure of the napkin.

Accordingly, it is the primary object of the present invention to provide a sanitary napkin which, during use, can easily conform to the shape of the perineal area (i.e., the anteroposterior curvature of the perineal area and the concave space between the buttocks) of the wearer and come into close contact therewith, regardless of individual variation in the shape of the perianeal area of females, which can exhibit satisfactorily high absorption for menstrual blood and prevent the leakage thereof, and which has a shape causing no reluctance on the part of the user.

Other objects of the present invention will become apparent from the description which follows.

In accordance with the present invention, there is provided a sanitary napkin comprising a layered structure including at least an absorbent material for menstrual blood, a blood-impermeable material and a covering material, characterized in that at least one bending groove which, during use, can produce the cross-sectional shape conforming to the recess between the buttocks of the wearer is provided on at least one side of said napkin; two bending grooves which, during use, can produce the cross-sectional shape conforming to the perineal area of the wearer is provided on at least one side of said napkin, said bending grooves being disposed in both lateral regions of said napkin along the longitudinal edges thereof; and at least one bending groove which, during use, can produce the cross-sectional shape conforming to the recess between the buttocks of the wearer is provided on at least one side of said napkin, and two bending grooves which, during use, can produce the cross-sectional shape conforming to the perineal area of the wearer is provided on at least one identical side of said napkin as the former bending groove is provided, the latter bending grooves being disposed in both lateral regions of said napkin along the longitudinal edges thereof.

BRIEF DESCRIPTION OF THE DRAWINGS

The accompanying drawings illustrate several examples of the sanitary napkin of the present invention that is provided with at least one bending groove which, during use, can produce a configuration conforming to the shape of the perineal area of the wearer.

Fig. 1 is a plan view of an exemplary sanitary napkin in which three bending grooves extending in the longitudinal direction are provided in its posterior region to be applied to the posterior part of the perineal area (i.e., the buttocks) of the wearer;

- 5 -

0136524

Fig. 2 is a cross-sectional view, taken along line A-A', of the sanitary napkin of Fig. 1 and Fig. 3 is a cross-sectional view thereof in use, illustrating its posterior region which assumes a cross-sectional shape conforming to the recess between the buttocks;

Fig. 4 is a plan view of the sanitary napkin of Fig. 1 in which two additional bending grooves are provided in both lateral regions thereof;

Fig. 5 is a cross-sectional view, taken along line B-B', of the sanitary napkin of Fig. 4 in use, illustrating its central portion which is curved toward the perineal area of the wearer under the influence of the rise of its posterior region as illustrated in Fig. 3 and comes into close contact therewith;

Figs. 6 and 7 are plan views of other exemplary sanitary napkins embodying the present invention; and

Figs. 8 to 10 are plan views of sanitary napkins in which at least one piece of pressure-sensitive adhesive tape is provided on the side opposite to that facing the perineal area of the wearer.

## DETAILED DESCRIPTION OF THE INVENTION

The term "bending groove" as used herein means any groove that has the form of a straight line, a curved line, a bent line, a dotted line or a combination thereof and can be formed by pressing a surface of a sanitary napkin with a stamping die having a raised pattern in the form of a straight line, a curved line, a bent line, a dotted line or a combination thereof.

In the sanitary napkin of the present invention that includes at least an absorbent material for menstrual blood, a blood-impermeable material and a covering material, at least one bending groove as defined above may be provided on one or both sides of the layer of the absorbent material, the integrated layer of the absorbent material and the blood-impermeable material, or the whole structure of the napkin.

Moreover, such a bending groove or grooves may be provided in any form of sanitary napkin, regardless of the shape of the napkin or the type of the materials constituting it.

The present invention will be more specifically described hereinbelow with reference to the accompanying drawings.

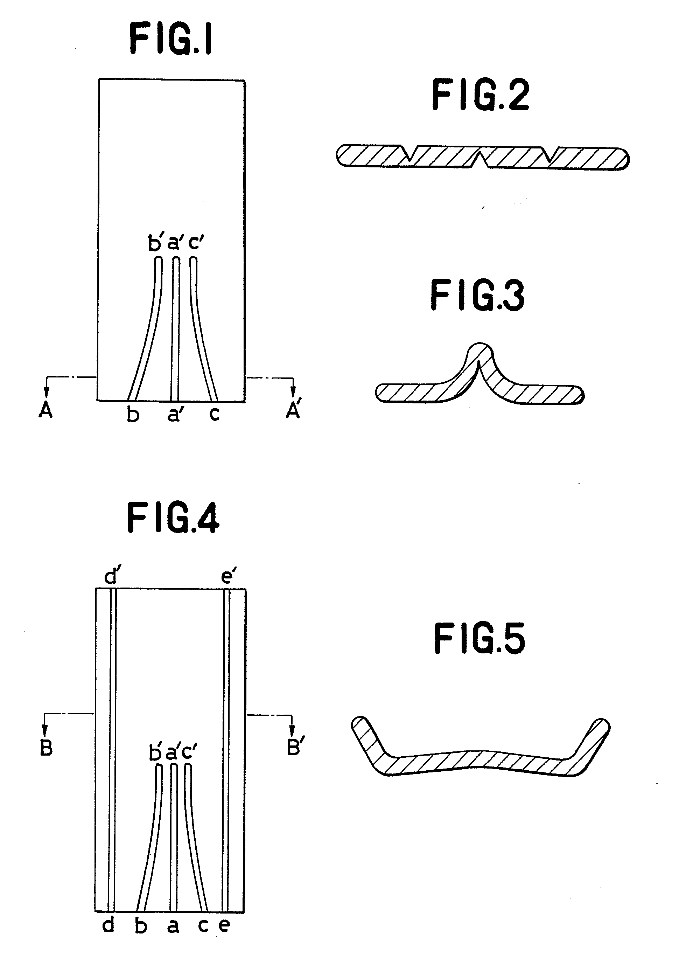

Fig. 1 illustrates an exemplary sanitary napkin in which a rectilinear groove a-a' disposed along its longitudinal axis and two curvilinear grooves b-b' and c-c' located on both sides thereof are provided in a surface of the posterior region of the napkin, i.e., the region which, during use, will be applied to the posterior part of the perineal area (or the buttocks) of the wearer. During use, the sanitary napkin provided with these grooves are easily bent at the groove a-a' under the lateral pressure exerted by the thighs of the wearer and the upward pressure exerted by the undergarment, so that the napkin rises toward the perineal area of the wearer to assume the cross-sectional shape of " ⌒ " as illustrated in Fig. 3 and further, the cross-sectional shape of " ⌣⌢⌣ " or " ⌣⌣ ". Thus, the posterior region of the sanitary napkin provided with the above-described grooves fits closely to the recess between the buttocks of the wearer, whereby the napkin is fixed in place. In addition, over its posterior to anterior region, the sanitary napkin is curved toward the perineal area of the wearer in a bilaterally symmetrical manner with respect to the extension of the groove a-a' (i.e., the longitudinal axis of the napkin) and comes into close contact therewith.

Furthermore, the upward pressure exerted by the undergarment causes the longitudinal section of the sanitary napkin to conform to the anteroposterior curvature of the perineal area of the wearer and comes into close contact therewith.

From the viewpoint of fitting to the perineal area, it is preferable that the posterior region of the sanitary napkin in which the above-described grooves are provided should occupy about 30% to 40% of the entire surface of the napkin. Although the posterior region of the sanitary napkin illustrated in Fig. 1 is provided with three grooves, only one groove a-a' can also produce a cross-sectional shape conforming to the recess between the buttocks of the wearer. Thus, it is to be understood that no particular restriction is imposed on the number of grooves provided according to the present invention.

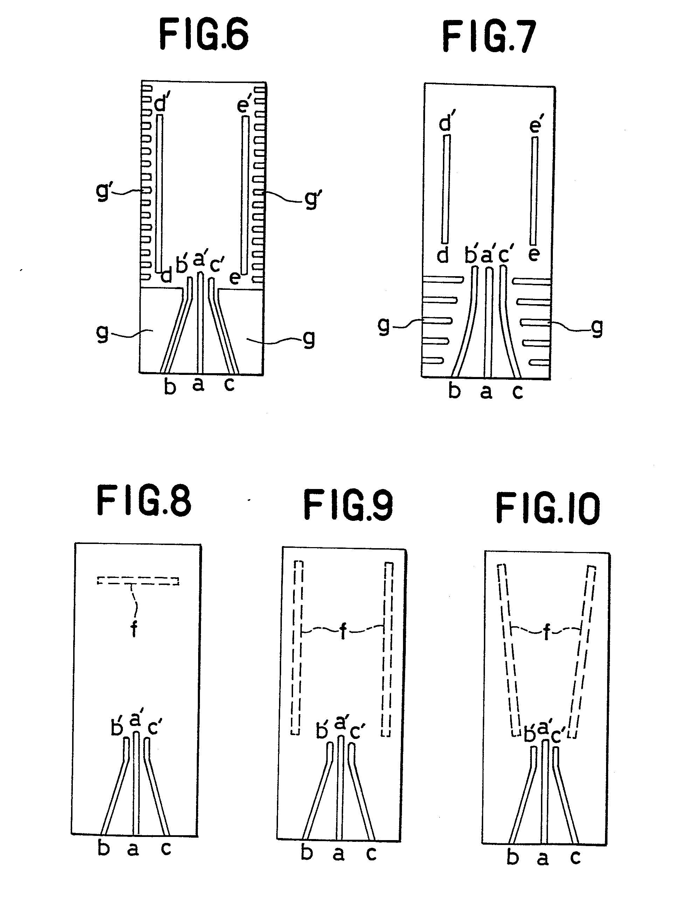

In the sanitary napkin of the present invention, additional bending grooves d-d' and e-e' may be provided in both lateral regions of the napkin, as illustrated in Fig. 4. During use, these grooves cooperate with the rising effect of the above-described groove a-a' and thereby cause the intermediate region of the napkin to assume a cross-sectional shape of " ‿⌄ " as illustrated in Fig. 5 and further the cross-sectional of " ⌣⌐ ", " ⌐⌣ " or " ⌣⌣ ". More specifically, the central portion of the napkin is curved toward the perineal area of the wearer and brought into close contact therewith. At the same time, the portions outside the grooves d-d' and e-e' rise under the lateral pressure exerted by the thighs of the wearer and thereby forms barriers for preventing the lateral leakage of menstrual blood.

Moreover, in the sanitary napkin of the present invention, auxiliary grooves or indentations may also be provided in proximity to the above-described bending grooves and in perpendicular relationship therewith, as illustrated in Fig. 6 and 7. During use, these auxiliary grooves or indentations give resistance to the force exerted on the surfaces of the napkin, so that unwanted bending of the napkin is prevented to ensure the desired bending thereof at each of the above-described bending grooves.

Similarly to the above-described bending grooves, these auxiliary grooves or indentations may be provided on one or both sides of the layer of the absorbent material, the integrated layer of the absorbent material and the blood-impermeable material, or the whole structure of the napkin, regardless of the shape of the napkin or the type of the materials constituting it. They can be formed as linear or dashed indentations having the form of one or more straight lines, curved lines, bent lines, dotted lines or combinations thereof. In addition, these indentations may also include planar indentations g as illustrated in Fig. 6.

Furthermore, in the sanitary napkin of the present invention, at least one piece f of pressure-sensitive adhesive tape may be provided at any desired position on the side opposite to that facing the perineal area of the wearer, as illustrated in Figs. 8 to 10, for the purpose of fixing the napkin in place during use. Thus, the side of the napkin provided with the above-described adhesive tape is attached to the undergarment, so that the napkin is more assuredly brought into close contact with the perineal area of the wearer.

As described above, the present invention provides a sanitary napkin provided with at least one bending groove which, during use, can produce a configuration conforming to the shape of the perineal area (i.e., the anteroposterior curvature of the perineal area and the concave space between the buttocks) of the wearer, thus allowing the napkin to fit the perineal area of the wearer and come into close contact therewith, regardless of individual variation in the shape of the perineal area.

In addition, since the sanitary napkin of the present invention can be obtained simply by providing a conventional sanitary napkin with the above-described bending groove or grooves without

- 11 -

altering its shape, the present invention is applicable to any form of sanitary napkin. Further, the sanitary napkin of the present invention has the advantage that it can be manufactured by the same mass production system and in the same manner of packaging as used in the manufacture of conventional sanitary napkins on an industrial scale.

The present invention is further illustrated by the example below. However, this example is not to be construed to limit the scope of the invention.

Example

Preparation of main body of sanitary napkin:

On the underside of a blood-absorbing layer comprising ground pulp was disposed a blood-absorbing and holding layer comprising a powdered hydrolyzate of starch-acrylonitrile graft polymer which is a highly absoptive high polymer and a sheet of water-absorbing paper made from pulp. Then, on the underside of this blood-absorbing and holding layer was disposed a blood-impermeable layer comprising a sheet of waterproofed paper made from pulp and a sheet of polyethylene laminated thereto. The resulting layered structure was covered with a thin non-woven fabric composed of hydrophobic polyester-polypropylene fibers to obtain a sanitary napkin of a flat, rectanguar shape.

Formation of bending grooves in napkin surfaces:

Using stamping dies which had previously been made, three grooves as illustrated in Fig. 1 were formed on both sides of the posterior region of the napkin comprising the layered structure obtained as above.

The products manufactured in the above-described manner were evaluated by asking female testers to use them during periods of menstruation. Thus, it was reported that they fitted the shape of the perineal area of the wearer comfortably and came into close contact therewith, resulting in no leakage of menstrual blood. Moreover, they caused no reluctance on the part of the user.

Z/2563

What is claimed is:

1. A sanitary napkin comprising a layered structure including at least an absorbent material for menstrual blood, a blood-impermeable material and a covering material, characterized in that at least one bending groove which, during use, can produce the cross-sectional shape conforming to the recess between the buttocks of the wearer is provided on at least one side of said napkin.

2. A sanitary napkin comprising a layered structure including at least an absorbent material for menstrual blood, a blood-impermeable material and a covering material, characterized in that two bending grooves which, during use, can produce the cross-sectional shape conforming to the perineal area of the wearer is provided on at least one side of said napkin, said bending grooves being disposed in both lateral regions of said napkin along the longitudinal edges thereof.

3. A sanitary napkin comprising a layered structure including at least an absorbent material for menstrual blood, a blood-impermeable material and a covering material, characterized in that at least one bending groove which, during use, can produce the cross-sectional shape conforming to the recess between the buttocks of the wearer is provided on at least one side of said napkin, and two bending grooves which, during use, can produce the cross-sectional shape conforming to the perineal area of the wearer is provided on at least one identical side of said napkin as the former bending groove is provided, the latter bending grooves being disposed in both lateral regions of said napkin along the longitudinal edges thereof.

4. A sanitary napkin as claimed in claim 1 wherein auxiliary indentations which, during use, aid in producing said cross-sectional shape are provided on at least one identical side of said napkin as said bending groove is provided.

5. A sanitary napkin as claimed in claim 2 wherein auxiliary indentations which, during use, aid in producing said cross-sectional shape are provided on at least one identical side of said napkin as said bending grooves are provided.

6. A sanitary napkin as claimed in claim 3 wherein auxiliary indentations which, during use, aid in producing said cross-sectional shape are provided on at least one identical side of said napkin as said bending grooves are provided, and auxiliary indentations which, during use, aid in producing said cross-sectional shape are provided on at least one identical side of said napkin as said bending grooves are provided.

7. A sanitary napkin as claimed in claim 1 wherein said bending groove is provided on at least one side of the posterior region of said napkin.

8. A sanitary napkin as claimed in claim 2 wherein said bending grooves are provided on at least one side of the intermediate or posterior region of said napkin.

9. A sanitary napkin as claimed in claim 1 or 7 wherein said bending groove is provided on at least one side of the layer of said absorbent material.

10. A sanitary napkin as claimed in claim 2 or 8 wherein said bending grooves are provided on at least one side of the layer of said absorbent material.

11. A sanitary napkin as claimed in claim 3 wherein said bending grooves are provided on at least one side of the layer of said absorbent material.

12. A sanitary napkin as claimed in any one of claims 1-3 wherein at least one piece of pressure-sensitive adhesive tape for fixing said napkin in place is provided on the side opposite to that facing the perineal area of the wearer.

# FIG.1

# FIG.2

# FIG.3

# FIG.4

# FIG.5

# FIG.6

# FIG.7

# FIG.8

# FIG.9

# FIG.IO

# EUROPEAN SEARCH REPORT

Application number

EP 84 11 0149

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| X | US-A-3 575 174 (MOGOR)<br>* Claim 1; figures 1, 3, 5, 6 * | 1 | A 61 F 13/18 |
| A | | 2 | |
| X | US-A-3 411 504 (GLASSMAN)<br>* Claim 1; figure 5 * | 1 | |
| A | | 2,3 | |
| A | US-A-4 067 336 (JOHNSON)<br>* Claim 1 * | 1 | |
| A | US-A-3 430 630 (MEGISON et al.)<br>* Claim 1 * | 1 | |
| A | US-A-3 343 543 (GLASSMAN)<br>* Claim 1 * | 1 | TECHNICAL FIELDS SEARCHED (Int. Cl.4)<br><br>A 61 F 13/00 |
| A | US-A-2 787 271 (CLARK) | | |
| A | DE-B-2 460 712 (KIMBERLY-CLARK CORP.) | | |

The present search report has been drawn up for all claims

| Place of search | Date of completion of the search | Examiner |
|---|---|---|
| BERLIN | 22-11-1984 | KANAL P K |